(19) Europäisches Patentamt
European Patent Office
Office européen des brevets

(11) Publication number: **0 035 742**
**B1**

(12) **EUROPEAN PATENT SPECIFICATION**

(45) Date of publication of patent specification: **30.05.84**

(21) Application number: **81101543.7**

(22) Date of filing: **04.03.81**

(51) Int. Cl.$^3$: **C 07 H 15/04,** C 12 P 19/44 // A61K7/24 ,(C12P19/44, C12R1/465)

(54) **Homocitric acid oligoriboside derivatives, process for their manufacture and their use as preventives of dental caries.**

(30) Priority: **08.03.80 JP 29690/80**

(43) Date of publication of application: **16.09.81 Bulletin 81/37**

(45) Publication of the grant of the patent: **30.05.84 Bulletin 84/22**

(84) Designated Contracting States: **AT BE CH DE FR GB IT LI NL SE**

(56) References cited: **None**

(73) Proprietor: **BISEIBUTSU KAGAKU KENKYUKAI**
**3-14-23, Kamiosaki Shinagawa-ku**
**Tokyo (JP)**

(72) Inventor: **Okami, Yoshiro**
**4-18-14, Denenchofu**
**Ohta-ku Tokyo (JP)**
Inventor: **Yamada, Kazuhiko**
**3-22-22, Fujigaoka**
**Fujisawa City Kanagawa Pref. (JP)**
Inventor: **Takashio, Masachika**
**1-35-14, Komagome**
**Toshima-ku Tokyo (JP)**

(74) Representative: **Reuter, Johann-Heinrich, Dr.**
**HOECHST AKTIENGESELLSCHAFT Zentrale**
**Patentabteilung Postfach 80 03 20**
**D-6230 Frankfurt/Main 80 (DE)**

**EP 0 035 742 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to preventive of dental caries containing homocitric acid oligoriboside and its salts together with their manufacturing processes. The structure of the homocitric acid oligoriboside derivative is shown as follows.

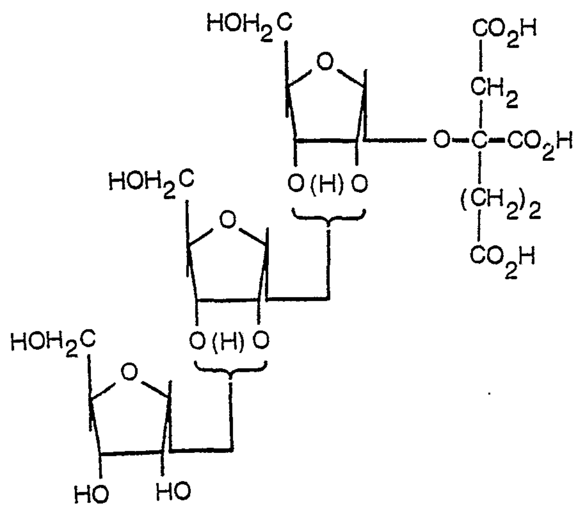

I

Each glycosidic linkage between ribose moieties is one of a ribosyl(1→2)-ribosyl or a ribosyl-(1→3)-ribosyl bond.

The homocitric acid oligoriboside described above is formed in the cultured broth of *Streptomyces* sp. MF980-CFI (FERM—P5430) and produced by the following method, for instance.

The organism is cultured in an usual medium consisting of carbon and nitrogen sources, inorganic ions and, if necessary, organic microelements, such as vitamins and/or amino acids. The homocitric acid oligoriboside formed and accumulated in the culture broth is extracted, separated and collected. As the carbon source in the medium, carbohydrates such as glycerol, glucose, maltose, sucrose, lactose, starch and dextrin can be used for the production. Especially, maltose or potato starch are suitable. Soy bean meal, peanut meal, cotton seed meal, dry yeast, peptone, meat extract, casein, corn steep liquor, nitrate nitrogen and ammonia nitrogen etc. can be used as the nitrogen source. Cotton seed meal or corn steep liquor are especially preferable. If necessary, the medium is further supplemented with magnesium, manganese, sodium, potassium, iron, calcium, chlorine, phosphate and/or sulfate ions. Aerobic cultivation by aeration, agitation or shaking and with an incubation temperature ranging from 25°C to 30°C are suitable for the production. Any other incubation temperature allowing the growth of the producer organism for the production of homocitric acid oligoriboside can be employed. Incubation of the culture is continued until the said substance is accumulated enough in the cultured broth. Usually, the incubation time is 2 to 7 days.

Microbiological properties of streptomyces sp. MF 980-CFI (FERM—P5430), is as follows. The strain was isolated from a soil collected around Lake Kawaguchi.

a) Morphology

Strain MF980-CFI grown well on agar media shows linear or spiral structure of aerial mycelia elongated from simple-branched, well elongate substrate mycelia. When matured, spore chains of 10—50 cylindrical spores (0.8 × 1.8 μm) are formed on top of the mycelia. Observations by electron microscopy revealed smooth surface spores and no spiny or hairy structure on spores. Neither flagella or sporangia observed. Consequently, it can be classified in typical *Streptomyces* sp.

b) Characteristics on various media

1) Sucrose-nitrate agar (27°C incubation):

White (white a) and poor aerial mycelia are formed above the substrate growth of yellowish brown color (2 db extra pastel series for hue, by Color Harmony Manual). No distinctive diffusible pigments are formed in the medium.

2) Glycerol-Asparagine Agar (27°C incubation):

Substrate growth shows weak reddish brown (5 lg, hue 5) color. White aerial mycelia first appear on the peripheral parts of the colony and gradually cover the whole parts. No distinctive soluble pigments are formed.

3) Starch Agar (27°C incubation):

Brownish white (5 ba, near gray series) aerial mycelia are formed above the orange (4 pe, hue 4) colored substrate growth. No distinctive soluble pigments are formed.

4) Tyrosine Agar (27°C incubation):

Substrate growth shows reddish brown (5 lg, hue 5) color. White (white a) aerial mycelia are formed on the peripheral part of the colony. Melanoid pigment is formed.

5) Nutrient Agar (27°C incubation):

Growth is poor and shows no distinctive color. Aerial mycelium formation is also very poor.

6) Yeast-Malt Agar (27°C incubation):

Grayish white aerial mycelia are formed above the brown (chm 6 ng, hue 6) substrate growth and gradually turn to purplish gray (chm 5 do near gray series). Brownish melanoid pigment is formed.

7) Oat Meal Agar (27°C incubation):

Brownish gray (chm 13 fe near gray series) aerial mycelia are formed above the reddish orange (chm 5 le hue 5) colored substrate growth. No distinctive soluble pigment is formed.

c) Physiological properties

1) Temperature range for growth is 14—33°C.

2) Starch is hydrolysed on Starch Agar.

3) Skin milk is peptonized but not coagulated.

4) Melanoid pigment is formed in Tyrosine Agar, Yeast-Malt Agar and Peptone-Yeast-Iron Agar media.

5) Nitrate is reduced.

6) Gelatin is not liquefied.

d) Utilization of carbon sources (Pridham and Gottlieb medium).

D-Glucose, L-arabinose, sucrose, D-xylose, inositol, mannitol, D-fructose and L-rhamnose are utilised, but raffinose is not.

The above properties show this strain typically belongs to genus Streptomyces and has characteristics such as formation of spiral structure on its aerial mycelia and formation of melanoid pigment.

Those properties resemble those of the following species, but there are some differences as follows.

1) Streptomyces griseoaurantiacus:

No melanoid pigment formation and very poor utilization of sucrose are different from those of strain MF980-CFI.

2) Streptomyces resistomycificus:

No pigment formation, utilization of raffinose and pH dependence of pigment color of substrate growth differ from those of strain MF980-CFI.

3) Streptomyces diastochromogenes:

No pigment formation on Yeast-Malt agar medium and utilization of raffinose show the difference between the species and strain MF980-CFI.

4) Streptomyces galbus;

Pigment formation on glycerol-asparagine agar, starch agar and oatmeal agar and very poor utilization of sucrose and rhamnose differ from strain MF980-CFI.

5) Streptomyces neyagawaensis:

Pigment formation on glycerol-asparagine agar, starch agar and oatmeal agar and utilization of raffinose are different from those of strain MF980-CFI.

6) Streptomyces bottoropensis:

Pigment formation on glycerol-asparagine agar and utilization of raffinose differ strain MF980-CFI from the species.

Since there is no known species showing the characteristics of this strain in the genus Streptromyces, the species of this strain is concluded as a new one.

We named this strain as *Streptomyces* sp. MF980-CFI and deposited at Fermentation Research Institute, Agency of Industrial Science and Technology, Ministry of International Trade and Industry as accession No. FERM—P5430.

The homocitric acid oligoriboside can be obtained from the fermented broth by the known methods, based on its acidic character above all, the basic anion exchangers are most favourable to adsorbe this material.

As the anion exchangers, for example, Amberlite® CG400 or CG-4B (Rhom and Haas Co.), Diaion® PA316 or WA30 (Mitsubishi Chemical Industries) or DEAE Sephadex® (Pharmacia) and others of $OH^-$, $Cl^-$ or $HCO_3^-$ form or those mixture form are applicable.

After washing the adsorbent with water, the adsorbed homocitric acid oligoriboside is eluted with solution of sodium hydroxide, ammonium bicarbonate or mineral salts with good yield. Usually sodium chloride (0.1—1 M) or ammonium bicarbonate (0.1—1 M) are employed.

As homocitric acid oligoriboside is practically not adsorbed to the cation exchanger, the cation exchangers are available to remove the basic impurities.

Homocitric acid oligoriboside derivative can be separated by the thin layer chromatography of cellulose (Avicel®) developed with the mixed solvent composed of nPropanol:3 M ammonium hydroxide (55:45 Vol) (Rf: 0.65). Based on this character, the effective purification of this substance is attained by employing the cellulose column chromatography with similar or related solvents.

Salt type of homocitric acid oligoriboside depends on the used solvents, for example, to form free form, sodium salt, lithium salt and so on.

As homocitric acid oligoriboside inhibits strongly dextran-sucrase (EC 2.41.5), it can be used as a medicine. Especially, for effective prevention of dental caries, it can be added to toothpaste solely or with other effective materials. Dental caries is the decay of teeth caused by demineralization of the enamel surface with organic acids produced by the bacteria adhered to tooth surface. The bacterial adherence to the smooth surface such as tooth was mediated by the sticky insoluble dextran produced from sucrose by dextransucrases of a certain group of oral Streptococcus. So the inhibition of dextransucrase means the prevention of dental caries.

For the purpose of dental caries prevention, homocitric acid oligoriboside derivative can be employed as an additive not only to the toothpaste but to chewing gum, soft drinks, candy and the other foodstuffs.

The detailed explanation will be described in the following illustrative procedures.

1) The production of homocitric acid oligoriboside.

*Streptomyces* sp. MF980-CFI (FERM—P5430) grown on the slant agar medium was inoculated to a medium composed of 1% maltose, 1% corn steep liquor, 1% Pharma media (Traders Oil Mill Company), (pH 6.2, 125 ml in 500 ml Sakaguchi flask). After the culture with shaking at 27°C for 5 days, the pH of the combined fermented broth (5 l) was adjusted to pH 8.0 with sodium hydroxide. Then the fermented broth was heat treated (60°C, 30 min) and filtered. The obtained filtrate (4.5 l) was passed through the column of Diaion® PA316 (OH form, 1.3 l, Mitsubishi Chemical Industries Co.), and the column was washed with 10 l of water.

The adsorbed homocitric acid oligoriboside was eluted with 1 M ammonium bicarbonate. The combined active fractions (1 l, yield 83%) was concentrated to 80 ml under reduced pressure.

The concentrate was gel filtrated by Sephadex® G-15 (1 l, Pharmacia Co.) with water as the eluting solvent. Each 50 ml-fraction was collected. The activity was found in No. 11—15 fractions. The combined active fraction (yield, 72%) was directly applied to the DEAE-Sephadex® A-25 column ($HCO_3^-$ form, 400 ml, Pharmacia Co.) and eluted with a linear gradient of 0.1—0.7 M ammonium bicarbonate. The activity was found in the fractions about 0.25 M of ammonium bicarbonate. The active fractions were combined (yield 64%) and the ammonium bicarbonate was removed under reduced pressure. The concentrate thus obtained was applied successively to DEAE-Sephadex® A-25 ($Cl^-$ form, 200 ml, Pharmacia Co.) and eluted with a linear gradient of 0.01 M-0.5 M sodium chloride.

The activity (yield 58%) was found in the fractions about 0.17 M of sodium chloride. The active fractions were combined and concentrated to 1 ml, and desalted by a long column of Sephadex® G-15 (30 ml) with water as eluting solvent (166 mg, yield 55%). After chromatography of silica gel (Silica AR CC-7, Mallinckrod Co.) with the mixed solvent (n Propanol:3 M ammonium hydroxide 70:30 Vol), the white crystals were obtained (90 mg, yield 49%).

2) Properties of the homocitric acid oligoriboside Na-salt.

1. White powder, mp 205—210°C (dec.).
2. Soluble in water; insoluble in organic solvents.
3. Hydrolysis of the homocitric acid oligoriboside gives riboses and homocitric acid.
4. Elemental analysis; Found: C 40.15, H 5.31%; Calcd. for $C_{22}H_{31}O_{19}Li_3 \cdot 2H_2O$: C 40.24, H 5.33%.
5. UV spectrum has no maximum.
6. Positive color reaction with phenol-sulfuric acid and orcinol-hydrochloride. Negative color reaction with ninhydrin, Nelson-Somogyi and diphenylamine-sulfuric acid.
7. The $^{13}C$ nmr spectrum in $D_2O$ is shown in Fig. 1.
8. The $^{1}H$ nmr spectrum in $D_2O$ is shown in Fig. 2.
9. The IR spectrum (KBr disk) is shown in Fig. 3.

These facts indicate that the homocitric acid oligoriboside derivative in this invention has the structure described previously.

3) Inhibitory activity to dextransucrase.

Dextransucrase was prepared as follows: *Streptococcus* mutans ATCC 27607 was inoculated to Brain Heart Infusion bouillon (Eiken Chemical Co.), and grown overnight at 37°C. The culture supernatant obtained by centrifugation was precipitated with ammonium sulfate at 50% saturation. The precipitated enzyme was desalted and purified with common procedures.

Inhibitory activity to dextransucrase was assayed as follows: 2.7 ml of substrate solution (0.3% sucrose, 0.04% sodium azide, 30 mM potassium chloride, 30 mM sodium chloride in 50 mM imidazole-HCl buffer (pH 6.8), 0.3 ml of test solution or water as control, and 50 $\mu$l of dextransucrase were mixed.

After incubation at 37°C for 14 hrs, the turbidity at 600 nm was measured against water as blank, and the percent inhibition was calculated from the following equation:

$$\text{Percent inhibition} = \frac{(\text{Turbidity of control}) - (\text{Turbidity of test})}{(\text{Turbidity of control})} \times 100$$

The definition of 1 unit of this material is the amount which gives 50% inhibition.

Fig. 4 shows the relation between the concentration of homocitric acid oligoriboside and the percent inhibition determined by the assay method described above.

4) Inhibition of *Streptococcus* mutans adherence. Heart Infusion bouillon (Difco) containing various concentrations of sucrose was autoclaved and added aseptically with various amounts of homocitric acid oligoriboside aqueous solution (filter-sterilized) or sterile water for control (Final volume is 3.0 ml and final concentrations were shown in Table 1). Each tube was inoculated 1 × $10^5$ CFU of *Streptococcus* mutans ATCC 27607 grown overnight at 37°C in Brain Hear Infusion bouillon (Difco) and incubated anaerobically in a candle jar at 37°C for 14 hr. The tube was positioned at an angle 30° from the horizontal. Nonadherent cells were removed by pouring off the broth and the test tube was washed three times with 3 ml of 50 mM phosphate buffer (pH 6.0). The adhered plaque (insoluble dextran and cellular aggregates formed as a result of insoluble dextran production) was suspended in 3 ml of phosphate buffer described above by mechanical stirring and by scraping the surface with a glass rod.

Turbidity of the suspension was measured at 600 nm. The results were shown in Table 1.

5) Explanations of figures.

Fig. 1 shows the $^{13}C$ nmr spectrum of homocitric acid oligoriboside Na-salt in this invention.

Fig. 2 shows the $^{1}H$ nmr spectrum of homocitric acid oligoriboside Na-salt in this invention.

Fig. 3 shows the IR spectrum of homocitric acid oligoriboside Na-salt in this invention.

Fig. 4 shows the relationship between concentration of the homocitric acid oligoriboside Na-salt and inhibition ratio (%) to dextransucrase activity.

TABLE 1

Inhibition of *Streptococcus mutans* Adherence

| Concentration ($\mu g$/ml) of Homocitric acid oligoriboside derivative (sodium salt) | Concentration (%) of sucrose | | | | | |
|---|---|---|---|---|---|---|
| | 0.5 | | 1.0 | | 2.0 | |
| | Turbidity (600 nm) | Percent inhibition | Turbidity (600 nm) | Percent inhibition | Turbidity (600 nm) | Percent inhibition |
| 0 | 0.617 | – | 0.595 | – | 0.502 | – |
| 50 | 0.510 | 17 | 0.340 | 43 | 0.333 | 33 |
| 100 | 0.249 | 60 | 0.173 | 71 | 0.132 | 73 |

**Claims for the Contracting States: BE CH DE FR GB IT LI NL SE**

1. The homocitric acid oligoriboside derivative of formula I and its salts,

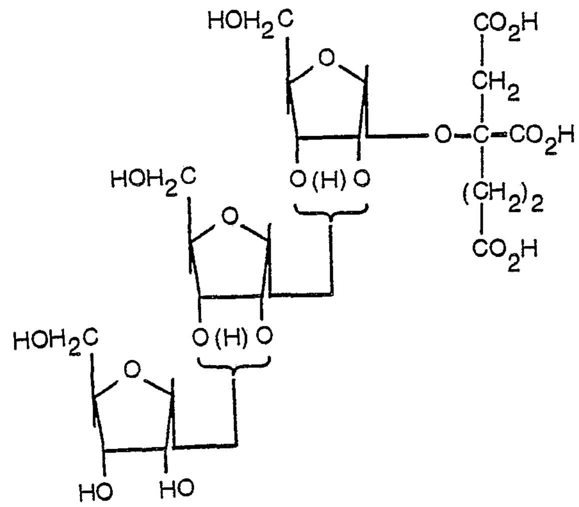

I

wherein each glycosidic linkage between ribose moieties is one of a ribosyl-(1→2)-ribosyl or a ribosyl-(1→3)-ribosyl bond, said compound having a melting point of 205—210°C (dec.), and being obtainable from cultured broth of streptomyces sp. MF 980 Cl (FERM—P 5430) by extraction, separation and collection.

2. The homocitric acid oligoriboside derivative of formula I, of claim 1 whenever obtained from cultured broth of Streptomyces sp. MF 980-CF1 (FERM—P 5430) by extraction, separation and collection thereof and its salts.

3. A process for obtaining a homocitric acid oligoriboside derivative according to claim 7, characterized in that a cultured broth of Streptomyces sp. MF 980-CF1 (FERM—P 5430) is extracted, the compound of formula I is separated and collected, and optionally converted into its salts.

4. A process according to claim 3 characterized in that said separation process is a combination of ion-exchange chromatography and gel filtration.

5. A compound according to any one of claims 1 and 2 for use of prevention of dental caries.

6. Preventives of dental caries containing a compound according to any one of claims 1 and 2.

**Claims for the Contracting State: AT**

1. A process for obtaining the homocitric acid oligoriboside derivative of formula I and salts thereof.

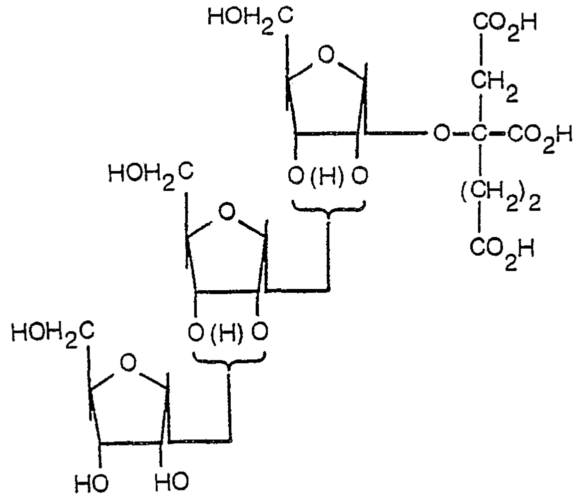

I

wherein each glycosidic linkage between ribose moieties is one of a ribosyl-(1→2)-ribosyl or a ribosyl-(1→3)-ribosyl bond, having a melting point of 205—210°C (dec.), characterized in that a cultured broth

of Streptomyces sp MF 980-CF1 (FERM—P 5430) is extracted, the compound of formula I is separated and collected, and optionally converted into its salts.

2. A process according to claim 1 characterized in that said separation process is a combination of ion-exchange chromatography and gel filtration.

3. A process according to any one of claims 1 and 2 for obtaining a compound of formula I for use of prevention of dental caries.

4. Preventives of dental caries containing the compound of formula I of claim 1 or its salts, said compound having a melting point of 205—210°C (dec.), and being obtainable from cultured broth of Streptomyces sp. MF 980-CF1 (FERM—P 5430) by extraction, separation and collection.

**Patentansprüche für die Vertragsstaaten: BE CH LI DE FR GB IT NL SE**

1. Homozitronensäureoligoridbosidderivat der Formel I

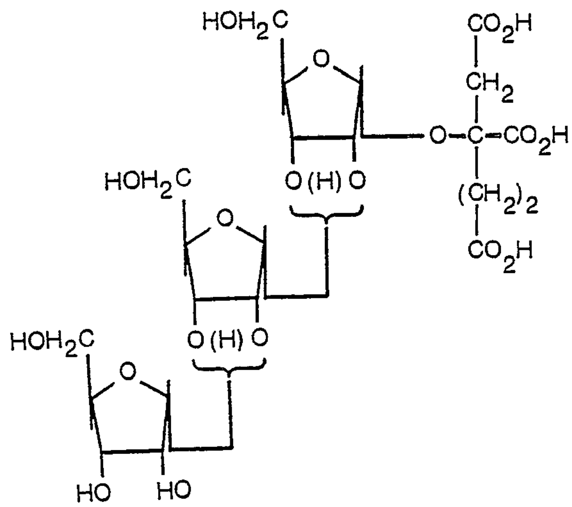

I

worin die einzelnen Glykosidbindungen zwischen den Riboseeinheiten Ribosyl (1—2)-Ribosyl oder Ribosyl ($\frac{1}{2}$—3)%Ribosylbindungen sein können, mit einem Schmelzpunkt von 205—210°C., (Zers.) das aus einer Kulturbrühe von Streptomyces sp. MF980 CF1 (FERM—P 5430) durch Extraktion, Abtgrennung und Sammeln isoliert werden kann.

2. Homozitronensäureoligoribosidderivat der Formel I gemäß Anspruch 1, wenn aus einer Kulturbrühe von Streptomyces sp. MF980—CF1 (FERM—P 5430) durch Extraktion, Abtrennung und Sammeln isoliert, sowie seine Salze.

3. Verfahren zur Gewinnung eines Homozitronensäureoligoribosidderivats gemäß Anspruch 1, dadurch gekennzeichnet, daß eine Kulturbrühe von Streptomyces sp. MF980-CF1 (FERM—P 5430) extrahiert wird, die Verbindung der Formel I davon abgetrennt und gesammelt wird und gegebenenfalls in ihre Salze überführt wird.

4. Verfahren gemäß Anspruch 3, dadurch gekennzeichnet, daß es sich bei dem Trennverfahren um eine Verbindung von Ionenaustauscherchromatographie und Gelfiltrierung handelt.

5. Verbindung gemäß einem der Ansprüche 1 und 2 zur prophylaktischen Anwendung gegen Karies.

6. Prophylaktische Mittel gegen Karies enthaltend eine Verbindung gemäß einem der Ansprüche 1 und 2.

**Patentansprüche für den Vertragsstaat: AT**

1. Verfahren zur Gewinnung des Homozitronensäureoligoribosidderivats der Formel I

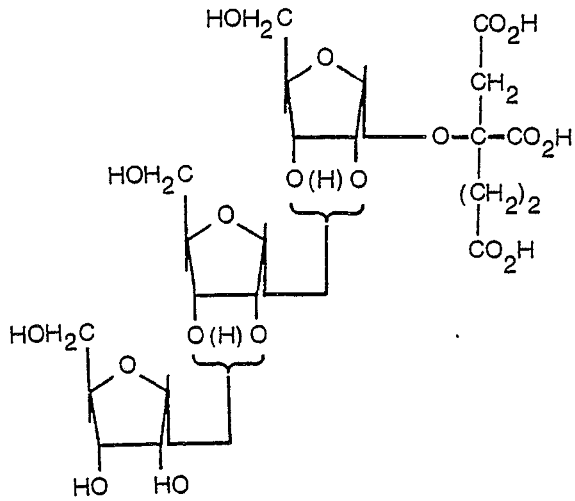

I

worin die einzelnen Glykosidbindungen zwischen den Riboseeinheiten Ribosyl (1→2)-Ribosyl oder Ribosyl (1→3)-Ribosylbindungen sein können, sowie seiner Salze, mit einem Schmelzpunkt von 205—210°C (Zers.), dadurch gekennzeichnet, daß eine Kulturbrühe von Streptomyces sp. MF980 CF1 (FERM—P 5430) extrahiert wird, die Verbindung der Formel I davon abgetrennt und gesammelt wird und gegebenenfalls in ihre Salze überführt wird.

2. Verfahren gemäß Anspruch 1, dadurch gekennzeichnet, daß der obige Trennungsprozeß eine Verbindung von Ionenaustauscherchromatographie und Gelfiltrierung ist.

3. Verfahren gemäß einem der Ansprüche 1 und 2 zur Gewinnung einer Verbindung der Formel I zur prophylaktischen Anwendung gegen Karies.

4. Prophylaktische Mittel gegen Karies enthaltend eine Verbindung der Formel I gemäß Anspruch 1, die einen Schmelzpunkt von 205—210°C (Zers.) hat und aus der Kulturbrühe von Streptomyces sp. MF980-CF1 (FERM—P5430) durch 35 Extraktion, Abtrennung und Sammeln gewonnen werden kann, oder ihre Salze.

**Revendications pour les Etats contractants: BE CH LI DE FR GB IT NL SE**

1. Le dérivé de l'acide homocitrique oligoriboside de formule I et ses sels,

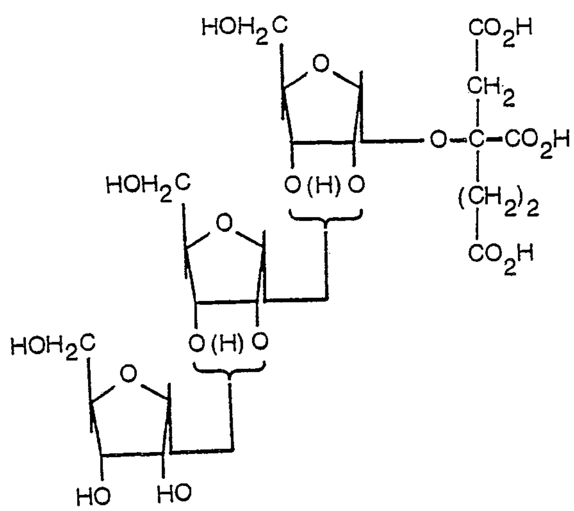

I

dans laquelle formule:
chaque liaison glycosidique entre des unités ribose est une des liaisons ribosyl-(1→2)-ribosyle ou

ribosyl-(1→3)-ribosyle, ledit composé ayant un point de fusion de 205—210°C (décomposition) et pouvant être obtenu à partir d'un bouillon de culture de Streptomyces sp. MF980-CF1 (FERM—P5430) par extraction, séparation et récolte.

2. Le dérivé de l'acide homocitrique oligoriboside de formule I selon la revendication 1, lorsqu'il est obtenu à partir d'un bouillon de culture de Streptomyces sp. MF 980-CF1 (FERM—P5430) par extraction, séparation et récolte du dérivé, et ses sels.

3. Un procédé d'obtention d'un dérivé d'acide homocitrique oligoriboside selon la revendication 1, caractérisé en ce qu'on extrait un bouillon de culture de Streptomyces sp. MF 980-CF1 (FERM—P5430), on sépare et on recueille le composé de formule I et éventuellement on le convertit en ses sels.

4. Procédé selon la revendication 3, caractérisé en ce que le procédé de séparation est une combinaison de chromatographie par échange d'ions et de filtration sur gel.

5. Composé selon l'une quelconque des revendications 1 et 2, pour utilisation pour la prévention des caries dentaires.

6. Agent de prévention des caries dentaires contenant un composé selon l'une quelconque des revendications 1 et 2.

**Revendications pour l'Etat contractant: AT**

1. Procédé d'obtention du dérivé de l'acide homocitrique oligoriboside de formule I et de ses sels,

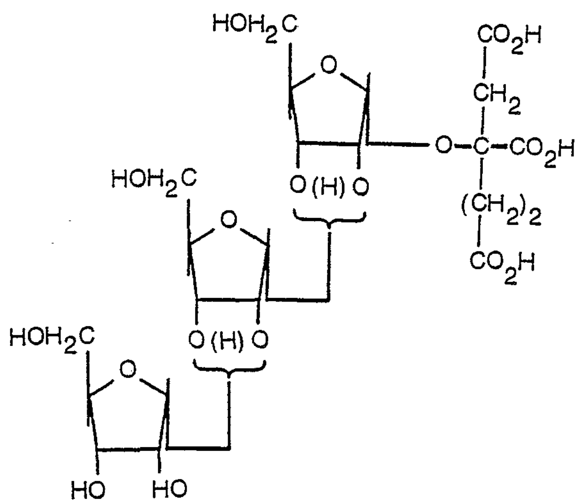

I

dans laquelle formule:
chaque liaison glycosidique entre des unités ribose est une liaison ribosyl-(1→2)-ribosyle ou ribosyl-(1→3)-ribosyle, ayant un point de fusion de 205 à 210°C (décomposition), caractérisé en ce qu'on extrait un bouillon de culture de Streptomyces sp. MF 980-CF1 (FERM—P5430), on sépare et on recueille le composé de formule I et on le convertit éventuellement en ses sels.

2. Procédé selon la revendication 1, caractérisé en ce que le procédé de séparation est une combinaison de chromatographie par échange d'ions et de filtration sur gel.

3. Procédé selon l'une quelconque des revendications 1 et 2, pour l'obtention d'un composé de formule I pour utilisation dans la prévention des caries dentaires.

4. Agent de prévention des caries dentaires contenant le composé de formule I selon la revendication 1 ou ses sels, ledit composé ayant un point de fusion de 205—210°C (décomposition) et pouvant être obtenu à partir d'un bouillon de culture de Streptomyces sp. MF 980-CF1 (FERM—P5430) par extraction, séparation et récolte.

FIG. 1

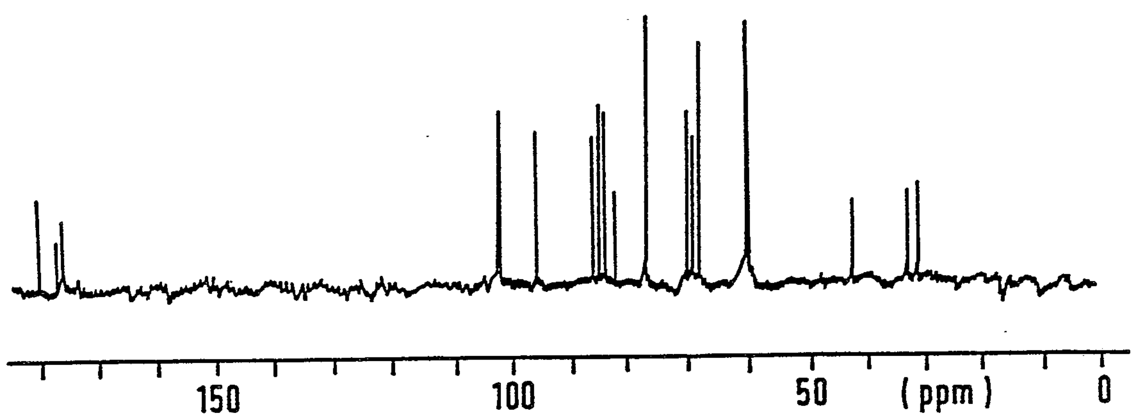


FIG. 2

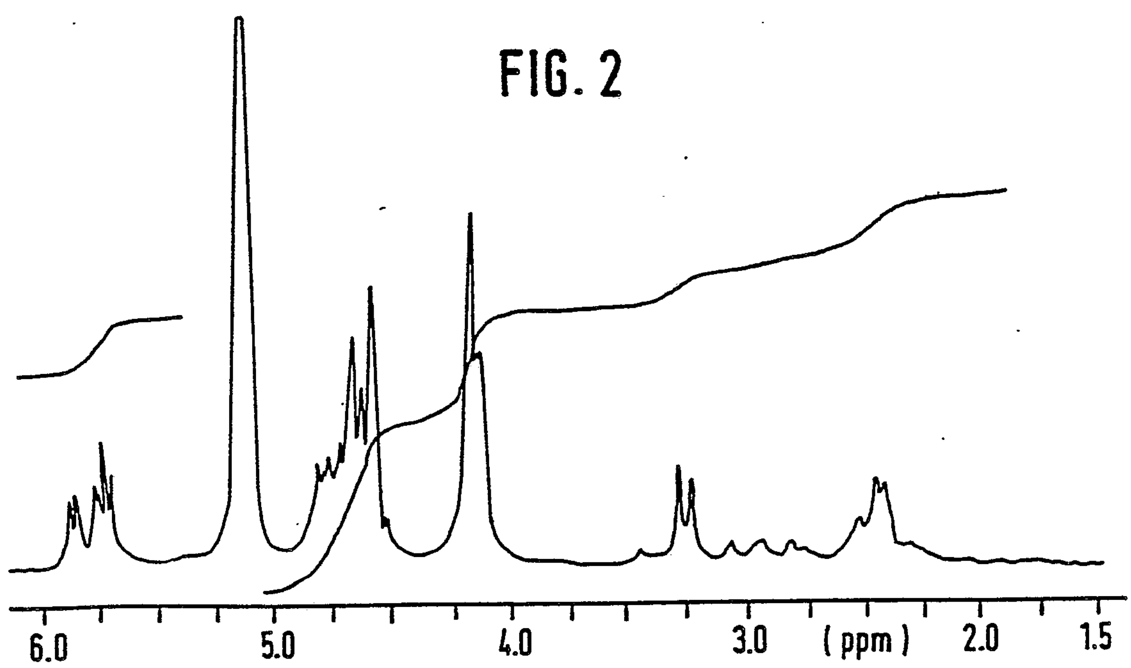

FIG. 3

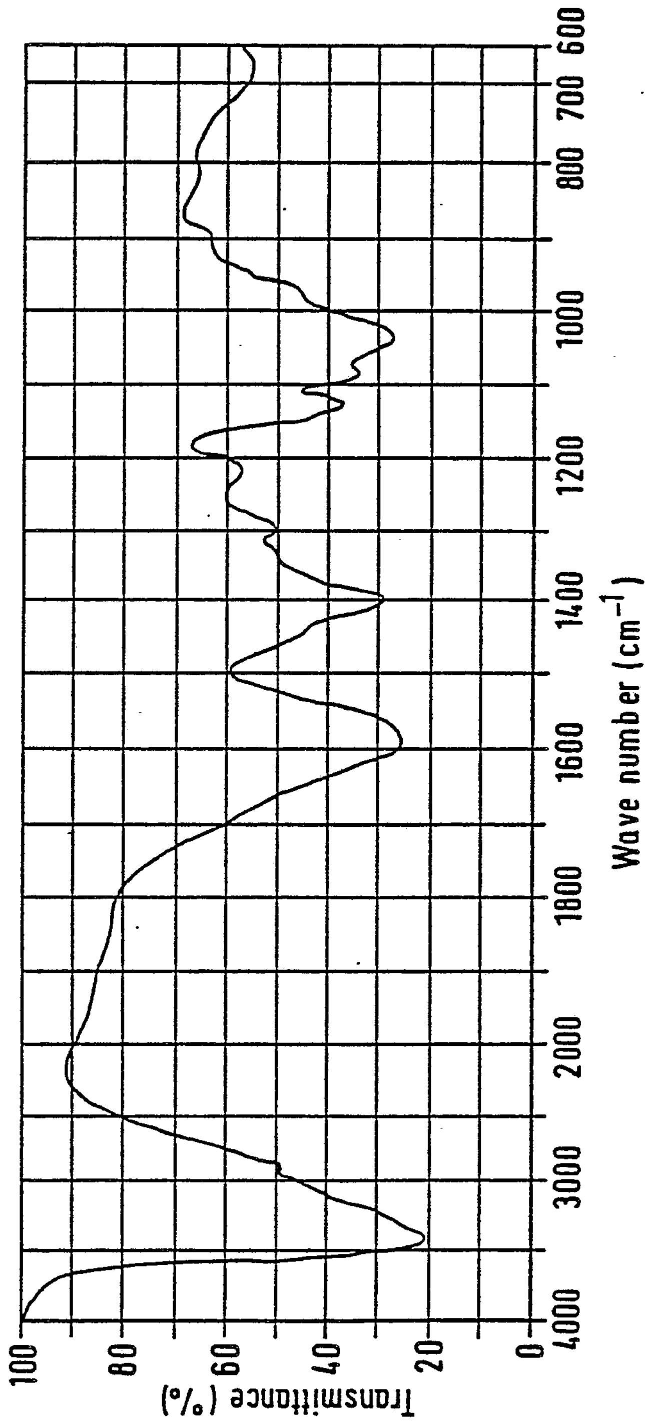
Transmittance (%)
100
80
60
40
20
0
4000
3000
2000
1800
1600
1400
1200
1000
800
700
600
Wave number (cm$^{-1}$)

FIG. 4

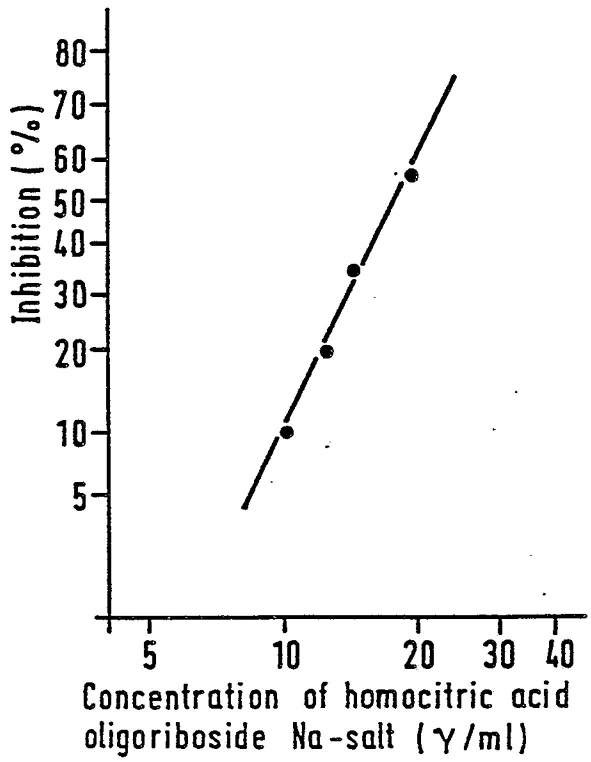
Inhibition (%)
80
70
60
50
40
30
20
10
5
5
10
20
30
40
Concentration of homocitric acid oligoriboside Na-salt (γ/ml)